**Europäisches Patentamt**

**European Patent Office**

**Office européen des brevets**

(11) Publication number: **0 117 032**

**B1**

(12)    **EUROPEAN PATENT SPECIFICATION**

(45) Date of publication of patent specification: **19.04.89**

(21) Application number: **84300118.1**

(22) Date of filing: **09.01.84**

(51) Int. Cl.⁴: **C 12 Q 1/26, C 12 Q 1/32**

(54) Rapid analysis of ethanol in body fluids.

(30) Priority: **12.01.83 CA 419293**
**22.07.83 US 516295**
**05.01.84 CA 443221**

(43) Date of publication of application:
**29.08.84 Bulletin 84/35**

(45) Publication of the grant of the patent:
**19.04.89 Bulletin 89/16**

(84) Designated Contracting States:
**BE CH DE FR GB IT LI LU NL SE**

(56) References cited:
**EP-A-0 019 937**
**DE-A-2 629 808**
**GB-A-1 351 547**
**GB-A-1 507 810**
**GB-A-1 528 309**
**US-A-3 493 467**
**US-A-3 917 453**
**US-A-4 202 938**
**US-A-4 351 899**

CHEMICAL ABSTRACTS, vol. 94, no. 5, 2nd
February 1981, page 416, no. 28827q,
Columbus, Ohio, US W.D. BOSTICK et al.:
"Hydroxylamine as a competitive inhibitor for

(73) Proprietor: **ALCOHOLISM AND DRUG ADDICTION RESEARCH FOUNDATION**
**33, Russell Street**
**Toronto Ontario, M5S 2S1 (CA)**

(72) Inventor: **Kapur, Bhushan Madhodass**
**2374, Canso Road**
**Oakville, ON L6J 5W6 (CA)**
Inventor: **Israel, Yedy**
**27, Blithfield Avenue**
**Willowdale, ON M2K 1X9 (CA)**

(74) Representative: **Votier, Sidney David et al**
**CARPMAELS & RANSFORD 43, Bloomsbury Square**
**London WC1A 2RA (GB)**

(56) References cited:
the kinetic enzymic assay of ethanol in
relatively concentrated solutions"

CHEMICAL ABSTRACTS, vol. 97, no. 3, 19th
July 1982, page 342, no. 19681x, Columbus,
Ohio, US A. PRYOR et al.: "Purification of maize
alcohol dehydrogenase and competitive
inhibition by pyrazoles"

Courier Press, Leamington Spa, England.

# EP 0 117 032 B1

**Description**

The present invention relates to the quantitative and qualitative determination of alcohol in biological fluids such as urine, saliva, serum or whole blood.

The effect of alcohol on human beings is a significant problem from both a social and medical viewpoint, the consequences of the impaired behavior of vehicle operators and of persons who consume excessive amounts of alcohol is increasingly being recognized as antisocial behaviour. Both for medical and social purposes it is important to obtain a fast accurate measurement of the blood alcohol level in a subject.

It is also important to identify the alcohol which causes the impairment. Alcohols other than ethanol may cause permanent damage unless the subject is treated promptly.

At the present time, various techniques are available for this purpose but such techniques necessitate the use of equipment which is expensive, cumbersome, not sufficiently accurate, or takes an unacceptable amount of time to determine the alcohol concentration.

It is therefore an object of this invention to provide an inexpensive, quick and reliable means of determining directly and accurately the alcohol concentration in a biological fluid and a clinically significant identification of the alcohol.

In accordance with one aspect of the invention, a quantitative determination of the alcohol level in body fluids is provided; in another aspect a qualitative determination of the alcohol level in the body fluids is provided: and in yet another aspect a simultaneous quantitative and qualitative determination of the alcohol levels in the body fluids may be provided.

In accordance with the present invention, a solid composition effective for determining the presence of a lower alcohol in a biological fluid without dilution, comprises a solid carrier having the following constituents mixed effective amounts:

an enzyme for oxidising the alcohol in the presence of a reducible compound to produce a corresponding aldehyde and a reduction product;

a chromogen which reacts with the reduction product in the presence of an agent to produce a coloured compound indicative of an alcohol presence in said biological fluid;

a competitive inhibitor of the enzyme;

an agent for converting the chromogen to the coloured compound; and

a buffer to maintain a predetermined pH.

The solid carrier impregnated with the alcohol-detecting reagents is immersed in the undiluted body fluid and gives a visual indication of the information to be determined.

In a first embodiment, the solid composition comprises an enzyme which oxidises ethanol in the presence of a reducible compound to produce the corresponding aldehyde, acetaldehyde, and a reduction product; a reducible compound; a competitive inhibitor of the enzyme; a chromogen which reacts with the reduction product in the presence of an agent to produce a coloured compound indicative of the presence and amount of ethanol in the biological fluid; an agent for converting the chromogen to the coloured compound; and a buffer to maintain a pH predetermined pH. This system will react with ethanol but not with methanol so as to permit a differential determination between ethanol and the other lower alcohols.

The enzyme may comprise alcohol dehydrogenase (ADH) and the reducible compound may comprise the co-enzyme nicotinamide adenine dinucleotide (NAD).

The competitive inhibitor may be pyrazole; a halogenated pyrazole; methyl, ethyl, propyl, butyl or pentyl pyrazole or a suitable steroid.

The solid composition takes advantage of competitive inhibitors of alcohol dehydrogenase to enable higher ethanol concentrations to be determined than would be possible in the absence of a competitive inhibitor.

In its natural state alcohol dehydrogenase has a very high affinity for ethanol such that the enzyme reacts with ethanol at a maximal constant velocity even at very low concentrations of ethanol. Quantitation by this method is not possible if the concentration of ethanol exceeds 50 mg/l. Since quantitation in the range of 50—1600 mg/l or higher is required, it is necessary to reduce the affinity of alcohol dehydrogenase for ethanol. In the presence of a suitable concentration of the competitive inhibitor of alcohol dehydrogenase, the rate at which the enzyme oxidizes ethanol, and thus yields the colour reaction, is proportional to the concentration of ethanol in the said range. Pyrazoles substituted in position 4 are more active competitive inhibitors, such that the concentrations required in the support system are: pyrazole>halogenated pyrazole>methyl pyrazole>ethyl pyrazole>propyl pyrazole>butyl pyrazole>pentyl pyrazole. The concentrations of compatitive inhibitors other than pyrazole are calculated by multiplying the concentration of pyrazole used by the ratios $K_i$(inhibitor)/$K_i$(pyrazole), where $K_i$'s are the inhibitor constants for the alcohol dehydrogenase reaction.

The chromogen may comprise a tetrazolium salt such as iodonitrotetrazolium chloride (INT), thiazolyl blue tetrazolium bromide (MTT) or nitroblue tetrazolium chloride (NBT). Alternatively, the chromogen may comprise 2-6-dichlorophenol indophenol, methylene blue or other suitable dyes or combinations thereof.

The agent for converting the chromogen to the coloured indicator compound may be diaphorase, phenazine or thiazine.

2

The buffer may comprise phosphate, pyrophosphate buffer or tris (hydroxymethyl) aminomethane buffer, commonly known as TRIS buffer.

The solid composition of the first embodiment may optionally contain an acetaldehyde trapping compound such as semicarbazide and a sulphydryl containing compound such as dithioerythritol to protect the enzyme.

In accordance with a second embodiment, the solid composition comprises an enzyme which oxidises a lower alcohol in the presence of the free oxygen contained in the biological fluid to produce the corresponding aldehyde and the reduction product, hydrogen peroxide; a competitive inhibitor of the enzyme; a chromogen which reacts with hydrogen peroxide in the presence of an agent to produce a coloured compound indicative of the presence and amount of lower alcohol in the biological fluid; an agent for converting the chromogen to the coloured compound; and a buffer to maintain a predetermined pH.

The enzyme of the second embodiment may be alcohol oxidase and the competitive inhibitor may be urea. As in the first embodiment, use of a competitive inhibitor of the enzyme permits quantitative determination without dilution of the biological fluid. The chromogen may be guaiacol, o-tolidine or a benzidine derivative such as tetramethyl benzidine. The agent for conversion of chromogen to the coloured indicator compound may be peroxidase and the buffer may be as in the first embodiment.

The second embodiment may be employed for the detection of lower alcohols such as methanol and ethylene glycol as well as ethanol.

The solid carrier may have two discrete areas, one impregnated with the reagents of the first embodiment and one with those of the second embodiment. If both areas show colour change when exposed to a biological fluid, ethanol is present, whereas if only one area changes colour, methanol or another lower alcohol other than ethanol is present.

In accordance with a third embodiment, the solid composition comprises a constituent which, in the presence of water, produces hydrogen peroxide; a first enzyme which oxidises ethanol in the presence of hydrogen peroxide to give the corresponding aldehyde, acetaldehyde; a competitive inhibitor of the first enzyme; a nucleotide; a second enzyme which reduces the nucleotide in the presence of acetaldehyde; a chromogen which reacts with the reduced nucleotide in the presence of an agent to produce a coloured indicator compound; an agent for converting the chromogen to the coloured compound; and a buffer to maintain a predetermined pH.

The constituent which generates hydrogen peroxide in presence of water may be barium peroxide or barium hydroperoxide; the first enzyme may be catalase; its competitive inhibitor may be sodium formate; the nucleotide may be NAD; the second enzyme may be acetaldehyde dehydrogenase; the chromogen may be a tetrazolium salt; the agent for generating the coloured compound may be diaphorase and the buffer may be as in previous embodiments.

With respect to all the embodiments of the invention described, if the enzymes are not covalently immobilized then an inert protein or proteins such as gelatin or albumin may be added to stabilise the enzyme while serving to enhance the colours produced.

It will also be appreciated that surfactants such as polyoxy-ethylene-23-laurylether known by the trademark BRIJ-34, or its equivalent, may be employed.

The solid carrier may, for example, be a strip of paper or may be an inert powder material such as silica gel, kieselghur or microcrystalline cellulose. The inert powder may be compounded with the active ingredients and compressed by known methods into tablet form or may be placed in an open-ended tube depending upon the application.

In the embodiments described freeze drying has been employed to ensure the stability of the composition. It will of course be understood that other forms of drying such as accelerated convection, at suitable temperatures, may be employed or a combination of such techniques may be employed.

It will be apparent to those skilled in the art that the solid carrier may be coated to enhance the optical qualitites of the final colour formed.

A semi-permeable coating may also be provided on the impregnated carrier over the impregnant. Such coatings may be formed in a conventional manner from materials such as cellophane, cellulose acetate, cellulose butyrate, ethyl cellulose or methyl cellulose so as to permit the alcohol and water to pass through and react with the impregnant while screening or preventing the red cells and larger molecules such as proteins and haemoglobin from passing through.

Example 1

Solid compositions intended for determining ethanol concentration in urine were prepared as follows:

1. Preparation of 125 mM TRIS Buffer pH 8.2 containing 0.84 g/100 ml semicarbazide. To 1.514 g of TRIS, 0.84 g of semicarbazide were added and diluted to 100 ml with double distilled water (adjusting pH to 8.2 with HCl). At this point gelatin may be added. If gelatin is added the solution is brought to 37°C and 1.5 g of gelatin added.

2. Preparation of stock solution of 25 mM pyrazole. 0.17 g of pyrazole were dissolved in 100 ml double distilled water.

3. Preparation of reagents to be freeze dried:

(a) the following were weighed in a lyophilizing bottle wrapped with aluminum foil to protect from light:

3

| 1. Diaphorase | 0.170 g (766 Units) |
| 2. NAD | 0.477 g |
| 3. ADH | (5250 Units) |
| 4. INT | 0.200 g |
| 5. Albumin | 0.14 g |
| 6. BRIJ-35 | 0.10 g |
| 7. Dithioerythritol | 85 µl of 25 mM Stock Solution |

(b) To this lyophilizing bottle, 14 ml of Tris Buffer and 0.37 ml of 25 mM pyrazole were added.

(c) The solution was gently mixed. After 15 minutes, 120 strips of Whatman® filter paper #3 were added to the solution in a dark room. The size of the strip filter paper was 5 mm×55 mm.

(d) The strips were allowed to absorb the reagent for about 5 minutes and were shaken gently. Any excess fluid was removed to be further used in the next batch.

(e) The lyophilizing bottle containing the filter strips was placed in the freezer for at least 3 hours and was freeze dried overnight.

4. Preparation and storage of freeze dried strips:

(a) The freeze dried strip (5 mm×55 mm) was attached to a semi-rigid plastic strip (55 mm×60 mm) by a double sided adhesive tape.

(b) The strips were cut to give a total of 11 small strips (5×60 mm). At the end of each small strip there was consequently a lyophilized pad of 5 mm×5 mm which contained all the reagents.

(c) The small strips were stored in dark bottles under a desiccant in a freezer (−15°C) or refrigerator.


Example 2

Solid compositions intended for determining ethanol concentration in serum or plasma were prepared as follows:

1. Preparation of 75 mM TRIS Buffer pH 8.2 containing 0.84 g/100 ml semi-carbazide. To 0.908 g of TRIS, 0.84 g of semi-carbazide were added and diluted to 100 ml with double distilled water (adjusting pH to 8.2 with HCL). Gelatin may be added as in Step 1 of Example 1.

Steps 2 to 4 were the same as Example 1.


Example 3

Solid compositions intended for determining ethanol concentration in saliva were prepared as follows:

1. Preparation of 138 mM TRIS Buffer pH 8.0 containing 0.84 g/100 ml semi-carbazide. To 1.671 g of TRIS, 0.84 g of semi-carbazide were added and diluted to 100 ml with double distilled water (adjusting pH to 8.0 with HCL). Gelatin may be added as in Step 1 of Example 1.

Steps 2 to 4 were the same as in Example 1.


Example 4

Solid composition for determination of ethanol concentration in whole blood.

Steps 1 to 3 were same as Example 2.

Then, the freeze dried strips were dipped into a 1.2% ethyl cellulose solution in acetone. Acetone is then removed by drying in vacuum.

Step 4 same as Example 1.


Example 5

Solid compositions intended for determining ethanol presence in urine, serum or saliva when quantitation was not required were prepared as follows:

The steps of Example 1 were carried out, except that the pyrazole was omitted.

Those skilled in the art will recognize that the sensitivity of detection of different ethanol levels can be varied depending on the specific application desired by altering the relative concentrations of pyrazole and alcohol dehydrogenase.

In use, a solid composition in accordance with the invention and prepared, for example, as described in the foregoing Examples is dipped for 1—2 seconds in a fluid suspected of containing ethanol. The intensity of the pink to red colour that develops in the solid composition is proportional to the concentration of ethanol present in the fluid. After 1 minute, the colour developed is compared to a colour chart. While the colour continues to develop in time, it can be fixed by briefly placing the solid composition on top of a filter paper pad that has previously been soaked in 1.0 M pyrazole and dried or dipped in HCl. The solid composition can be used for precise quantitation of ethanol using spectrophotometric instruments.

In order to study the accuracy of the present invention, ethanol concentration was determined in sample fluids by two methods:

1. A total of 920 urine samples from various populations with different alcohol consumption habits were collected over a period of 8 weeks, and their ethanol concentrations measured both by a solid composition prepared in accordance with Example 1 and by spectrophotometric analysis. (T. P. Hadjiioannou, S. I. Hadjiioannou, et al. Automated Enzymic Determination of Ethanol in Blood, Serum, and Urine with a Miniature Centrifugal Analyzer. Clinical Chemistry, Vol. 22 (6), 802—805 (1976)). The spectrophotometric results were then converted to the solid composition scale as follows:

$$>1600 \ mg/L=6$$
$$1200{-}1600 \ mg/L=5$$
$$800{-}1200 \ mg/L=4$$
$$400{-}800 \ mg/L=3$$
$$200{-}400 \ mg/L=2$$
$$50{-}200 \ mg/L=1$$
$$50 \ mg/L=0.5$$
$$0 \quad =0$$

Correlation statistics for the solid composition and the spectrophotometric method were calculated on the total samples and were as follows:

| | |
|---|---|
| 920 is the number of X and Y values. (X) | $=ay+b$ |
| Mean of all solid composition values (X) | $=1.1353$ |
| Mean of all Spectrophotometric values (Y) | $=1.1951$ |
| Slope (a) | $=1.0248$ |
| Intercept (b) | $=0.0315$ |
| Correlation Coefficient (r) | $=0.9831$ |

2. A total of 580 serum samples of patients who were suspected of having taken drugs, including alcohol, were tested by a solid composition prepared in accordance with Example 2 and by gas chromatography (Barbara R. Manno., Joseph E. Manno. A Simple Approach to Gas Chromatographic Microanalysis of Alcohols in Blood and Urine by a Direct-Injection Technique. Journal of Analytical Toxicology. Vol. 2, 257—261, Nov—Dec (1978)).

The gas chromatographic (GC) results were converted to the solid composition scale as follows:

$$>1400 \ mg/L=5$$
$$1000{-}1400 \ mg/L=4$$
$$600{-}1000 \ mg/L=3$$
$$300{-}600 \ mg/L=2$$
$$50{-}300 \ mg/L=1$$
$$<50 \ mg/L=0.5$$
$$0 \quad =0$$

The data was then correlated and the results were as follows:

| | |
|---|---|
| 580 is the number X and Y values. | |
| Mean of all solid composition values (X) | $=1.928$ |
| Mean of all GC values (Y) | $=2.050$ |
| Slope (a) | $=1.037$ |
| Intercept (b) | $=0.054$ |
| Correlation Coefficient (r) | $=0.9875$ |

These correlation coefficients (0.9831 and 0.9875) and slopes (1.0248 and 1.037) indicate that the solid composition in accordance with the invention gives ethanol concentrations that are nearly identical to those given by the use of complex spectrophotometric or gas chromatographic methods.

Interference studies

Interference studies were carried out with the solid composition as prepared in Example 1.

# EP 0 117 032 B1

### TABLE 1
#### Drugs in vitro

| | |
|---|---|
| Acetone | Hydrochlorothiazide |
| Amitriptyline | Imipramine |
| Amobarabital | Isopropanol |
| Amphetamine | Methadone |
| Ascorbic Acid | Methamphetamine |
| Butalbital | Methanol |
| Chlordiazepoxide | Methyprylon |
| Chlorpromazine | Morphine |
| Cimethidine | Naloxone |
| Cocaine | Nortriptyline |
| Codeine | Oxazepam |
| Diazepam | PCP |
| Digoxin | Phenobarbital |
| Dimenhydrinate | Phenylpropanolamine |
| Diphenhydramine | Propoxyphene |
| Ephedrine | Propranolol |
| Ethchlorvynol | Quinine |
| Florazepam | Salicylate |
| | Secobarbital |

Drugs added in-vitro

To pooled urine previously shown to contain no ethanol by gas chromatographic procedures, drugs listed in Table 1 and ethanol were added such that the final concentrations of both the drug and ethanol in urine were 1 mg/ml. In the case of methanol, isopropanol and acetone, their final concentration was 3 mg/ml. The urine sample were tested with the solid composition as prepared in Example 1 in the following manner: (1) after the drug was added and (2) after ethanol was added to sample containing the drug. The visual colour change with each testing was recorded. A urine sample from the same pool, containing 1 mg/ml of ethanol was used as a reference.

### TABLE 2
#### Drugs in-vivo

| | |
|---|---|
| Amitriptyline | Ludiomil |
| Amoxapine | Meprotiline |
| Amphetamine | Methadone |
| Barbiturate | Methamphetamine |
| Benzodiazepine | Morphine |
| Carbamazepine | Nortriptyline |
| Chlorpromazine | Oxycodone |
| Cimetidine | Pentazocine |
| Codeine | Imipramine |
| Desimipramine | Perphenazine |
| Diphenhydramine | Phencyclidine |
| Doxepin | Pheniramine |
| Ephedrinee | Phenothiazine |
| Ethchlorvynol | Phenylpropanolamine |
| Florazepam | Phenytoin |
| Haloperidol | Propoxyphen |
| Hydrocodone | Quinidine |
| | Quinine |

Drugs in-vivo

166 Urine samples from various drug using populations were tested in the following manner:

1. Drug screening was done using thin layer chromatographic procedures to establish the drugs content of the urine. Table 2 lists various drugs found either individually or in various combinations. Most drugs usually appear with their metabolites (not listed in the table).

2. Urine was tested with the solid composition as prepared in Example 1. Visual colour change at 1 minute was recorded.

(a) If there was a positive reading then the urine was further analyzed with gas chromatographic procedures to establish the urine ethanol level.

(b) If the solid composition did not change colour then ethanol was added to the urine such that the

6

final concentration was 100 mg/100 ml and retested with the solid composition. The visual colour change was recorded at 1 minute.

For comparison, a blank urine sample containing 100 mg/100 ml of ethanol was issued as reference. Air samples were analyzed within the day of their receipt in the laboratory.

Results of interference studies
Drugs added in-vitro

Of the 37 different drugs tested none interferred with the colour, as measured visually, or gave positive ethanol readings if ethanol was absent. Urine containing methanol, isopropanol, or acetone did not give a positive reaction in the presence of ethanol.

Drugs in-vivo

Most of the urine samples had more than two drugs and their metabolite present. 42 Urine samples had ethanol as one of the components present. In 124 samples ethanol had to be added. Samples containing large amounts of drugs or many drugs (3 or more) generally gave a negative bias of 1+, i.e. 6+ by gas chromatography read 5+ visually. Invariably the endogeneous levels of ethanol in these urine levels were greater than 100 mg/100 ml.

Example 6

An impregnant for determining lower alcohols in serum, plasma or saliva using alcohol oxidase.

1. 75 mM TRIS buffer at pH 7.5 was prepared by dissolving 0.908 g of tris (hydroxymethyl) aminomethane in 100 ml of distilled water, the pH was adjusted with $NH_4OH$.

2. 0.12 g of gelatin was added to 10 ml of the above TRIS buffer. Gelatin was dissolved by warming the buffer solution in a warm water bath.

3. Preparation of the reagents to be freeze dried.

(a) The following were weighed in a lyophilizing bottle wrapped with aluminum foil.

| | | |
|---|---|---|
| 1. Brij-35 | 0.0714 gm | |
| 2. Albumin | 0.1 gm | |
| 3. Peroxidase | 0.0869 gm (4000 units) | |

To the above reagents 10 ml of the TRIS buffer containing gelatin was added and mixed gently. It was allowed to stand for 15 minutes.

(b) To the above reagent mixture 300 µl (1000 units) of alcohol oxidase was added. The solution was gently mixed.

(c) After 15 minutes 100 strips of Whatman Filter paper #3 were added to the solution. The size of the strip filter paper was 5 mm×50 mm. The strips were allowed to absorb the reagent for about 5 minutes and shaken gently. Any excess fluid was removed to be further used in the next batch.

(d) The strips were allowed to absorb the reagent for about 5 minutes and shaken gently. Any excess fluid was removed to be further used in the next batch.

(e) The lyophilizing bottle containing the filter strips was placed in a freezer for at least 3 hours and subsequently freeze dried overnight.

4. Colour changing compound. Depending on the colour change desired any one or combination of the following chemicals can be made in 10 ml of acetone.

| Colour changing compound | | Concentration | final colour |
|---|---|---|---|
| a) Guaiacol | 10 nM | 0.012 g/10 ml | brown |
| b) Tetramethylbenzidine | 7 nM | 0.018 g/10 ml | blue green |
| c) Syringaldazine | 12 nM | 0.045 g/10 ml | violet |
| d) o-Tolidine | 10 nM | 0.021 g/10 ml | green |
| e) Diaminofluorine | 5 nM | 0.0098 g/10 ml | blue |

5. Inhibitor. 0.15 g of urea (0.25 mM) is dissolved in minimal amount of water and added to the acetone solution (4) containing the colour changing compound.

6. The lyophilized strips as prepared in 3e were soaked in the acetone solution (5) and excess solution was decanted off. The strips were dried on a stream of dry nitrogen.

7. Preparation and storage of the freeze dried strips.

a) The strip (5 mm×50 mm) was attached to a semi-rigid plastic strip (50 mm×60 mm) by a double sided adhesive tape.

b) The strip was then cut to give a total 10 small strips (5 mm×60 mm). At the end of each small strip there was consequently a lyophilized pad of 5 mm×5 mm which contained all the reagents.

c) The small strips were stored in dark bottles under a dessicant in a refrigerator or a freezer.

Example 7

A solid composition for determining lower alcohols in blood using alcohol oxidase.

Steps 1—3 same as in Example 6.

# EP 0 117 032 B1

In step 4, in addition to the colour changing compound, ethyl cellulose is added to give a final concentration of 1.2% in acetone.

Steps 5—7 same as in Example 5.

Example 8

Solid composition for determining lower alcohols using alcohol oxidase when quantitation is not required.

Step 5, the addition of urea, is omitted from Example 6 or 7.

The solid composition as described in Examples 6, 7 and 8 reacts with ethanol as well as methanol and ethylene glycol. When urea is used as the inhibitor at the above concentrations of methanol and ethanol, linearity between 0—1600 mg/l is achieved.

Those skilled in the art will recognize that the sensitivity of detection of different alcohol levels can be varied depending on the specific application desired by altering the relative amounts of urea and alcohol oxidase.

In use, the solid composition in accordance with the invention and prepared, for example, as described in the foregoing Examples 6 and 7 is dipped for 1—2 seconds in the fluid suspected of containing a lower alcohol. After 1 minute the colour developed in the solid composition is compared to a colour chart. The intensity of the color that develops in the solid composition is proportional to the concentration of lower alcohol present in the fluid. When the fluid assayed is blood, the solid composition is either washed with a few drops of water or wiped with a tissue before comparing with the colour chart.

**Claims**

1. A solid composition effective for determining the presence of a lower alcohol in a biological fluid without dilution, comprising a solid carrier having the following constituents mixed in effective amounts:

an enzyme for oxidizing said alcohol in the presence of a reducible compound to produce a corresponding aldehyde and a reduction product;

a chromogen which reacts with said reduction product in the presence of an agent to produce a coloured compound indicative of an alcohol presence in said biological fluid;

a competitive inhibitor of said enzyme;

an agent for converting said chromogen to said coloured compound; and

a buffer to maintain a predetermined pH.

2. A solid composition as claimed in claim 1 further comprising a reducible compound which reacts with said lower alcohol in presence of said enzyme to produce said reduction product.

3. A solid composition as claimed in any one of the preceding claims wherein said enzyme is alcohol dehydrogenase.

4. A solid composition as claimed in claim 2 or 3 wherein said reducible compound is nicotinamide adenine dinucleotide.

5. A solid composition as claimed in any one of the preceding claims, further comprising an acetaldehyde trapping compound.

6. A solid composition as claimed in claim 5 wherein said acetaldehyde trapping compound is semicarbazide.

7. A solid composition as claimed in any one of the preceding claims, further including a sulphydryl compound.

8. A solid composition as claimed in any one of the preceding claims wherein said enzyme is alcohol dehydrogenase and said competitive inhibitor is selected from a pyrazole, a halogenated pyrazole, an alkyl pyrazole and a steroid.

9. A solid composition as claimed in any one of the preceding claims wherein said chromogen is selected from a tetrazolium salt, 2-6-dichlorophenol indophenol and methylene blue and said agent is selected from diaphorase, phenazine and thiazine.

10. A solid composition as claimed in any one of the preceding claims further including an inert protein.

11. A solid composition as claimed in claim 1 wherein said enzyme is alcohol oxidase.

12. A solid composition as claimed in claim 1 or 11 wherein said competitive inhibitor is urea.

13. A solid composition as claimed in claim 1 or 12 wherein said agent is peroxidase.

14. A solid composition as claimed in claim 1 or 13, wherein said chromogen is selected from guaiacol, o-tolidine and a benzidine derivative.

15. A solid composition as claimed in claim 11 or 14 further including an inert protein.

16. For use in the determination of alcohol in biological fluids, an inert carrier having a first discrete area impregnated with a first alcohol-determining impregnant and a second discrete area impregnated with a second alcohol-determining impregnant, said first impregnant comprising, in effective amounts, nicotinamide adenine dinucleotide, alcohol dehydrogenase, a competitive inhibitor of alcohol dehydrogenase, a chromogen reactive in the presence of an agent chosen from diaphorase, phenazine and thiazine, one of diaphorase, phenazine or thiazine, an acetaldehyde trapping compound, a buffer to maintain a predetermined pH and an enzyme stabilising compound; and said second impregnant

8

comprising, in effective amounts, alcohol oxidase, a competitive inhibitor of alcohol oxidase, a chromogen reactive in the presence of peroxidase, peroxidase, a buffer to maintain a predetermined pH and an enzyme stabilising compound.

**Patentansprüche**

1. Feste Zusammensetzung, die wirksam ist zur Bestimmung der Anwesenheit eines niederen Alkohols in einer biologischen Flüssigkeit ohne Verdünnung, umfassend einen festen Träger, der die folgenden Bestandteile in wirksamen Mengen gemischt enthält:

ein Enzym zum Oxidieren des Alkohols in Gegenwart einer reduzierenden Verbindung unter Bildung eines entsprechenden Aldehyds und eines Reduktionsproduktes,

ein Chromogen, das mit dem Reduktionsprodukt in Gegenwart eines Agens unter Bildung einer Farbverbindung reagiert, die die Anwesenheit von Alkohol in der biologischen Flüssigkeit anzeigt,

einen kompetitiven Inhibitor für das Enzym,

ein Agens zum Umsetzen des Chromogens zur Farbverbindung und

einen Puffer zum Aufrechterhalten eines vorbestimmten pH-Wertes.

2. Feste Zusammensetzung nach Anspruch 1, weiter umfassend eine reduzierbare Verbindung, die mit dem niederen Alkohol in Gegenwart des Enzymes unter Ausbildung des Reduktionsproduktes reagiert.

3. Feste Zusammensetzung nach einem der vorhergehenden Ansprüche, wobei das Enzym eine Alkoholdehydrogenase ist.

4. Feste Zusammensetzung nach Anspruch 2 oder 3, worin die reduzierbare Verbindung Nikotinamid-adenindinukleotid ist.

5. Feste Zusammensetzung nach einem der vorhergehenden Ansprüche, die zusätzlich eine Acetaldehyd abfangende Verbindung umfaßt.

6. Feste Zusammensetzung nach Anspruch 5, wobei die Acetaldehyd abfangende Verbindung Semicarbazid ist.

7. Feste Zusammensetzung nach einem der vorhergehenden Ansprüche, die darüberhinaus eine Sulfhydrylverbindung enthält.

8. Feste Zusammensetzung nach einem der vorhergehenden Ansprüche, wobei das Enzym Alkoholdehydrogenase ist und der kompetitive Inhibitor ausgewählt ist aus einem Pyrazol, einem halogenierten Pyrazol, einem Alkylpyrazol und einem Steroid.

9. Feste Zusammensetzung nach einem der vorhergehenden Ansprüche, wobei das Chromogen ausgewählt ist aus einem Tetrazoliumsalz, 2-6-Dichlorphenol, Indophenol und Methylenblau und das Agens ausgewählt ist aus Diaphorase, Phenazin und Thiazin.

10. Feste Zusammensetzung nach einem der vorhergehenden Ansprüche, die darüberhinaus ein inertes Protein enthält.

11. Feste Zusammensetzung nach Anspruch 1, wobei das Enzym Alkoholoxidase ist.

12. Feste Zusammensetzung nach Anspruch 1 oder 11, wobei der kompetitive Inhibitor Harnstoff ist.

13. Feste Zusammensetzung nach Anspruch 1 oder 12, wobei das Agens Peroxidase ist.

14. Feste Zusammensetzung nach einem der Ansprüche 1 oder 13, worin das Chromogen ausgewählt ist aus Guajacol, o-Toluidin und einem Benzidinderivat.

15. Feste Zusammensetzung nach einem der Ansprüche 11 oder 14, die zusätzlich ein inertes Protein enthält.

16. Verwendung bei der Bestimmung eines Alkohols in einer biologischen Flüssigkeit von einem inerten Träger mit einem ersten diskreten Bereich, der mit einem ersten Alkohol-bestimmenden Imprägnierungsmittel imprägniert ist und einem zweiten diskreten Bereich, der mit einem zweiten Alkohol-bestimmenden Imprägnierungsmittel imprägniert ist, wobei das erste Imprägnierungsmittel in wirksamen Mengen Nikotinamid-adenindinukleotid, Alkoholdehydrogenase, einen kompetitiven Inhibitor für Alkoholdehydrogenase, ein Chromogen, das in Gegenwart eines Agens, ausgewählt aus Diaphorase, Phenazin und Thiazin reaktiv ist, eines von Diaphorase, Phenazin oder Thiazin, einer Acetaldehyd abfangenden Verbindung, einem Puffer zur Aufrechterhaltung eines vorbestimmten pH-Wertes und eine Enzym-stabilisierende Verbindung umfaßt und das zweite Imprägnierungsmittel in wirksamen Mengen Alkoholoxidase, einen kompetitiven Inhibitor für Alkoholoxidase, ein Chromogen, das in Gegenwart von Peroxidase reagiert, Peroxidase, einen Puffer zur Aufrechterhaltung eines vorbestimmten pH-Wertes und eine Enzym-stabilisierende Verbindung umfaßt.

**Revendications**

1. Composition solide efficace pour etablir la présence d'un alcool inférieur dans un fluide biologique sans dilution, comprenant un support solide dont les constituents suivants sont mélangés en quantités efficaces:

une enzyme servant à oxyder l'alcool en présence d'un composé réductible pour former un aldéhyde correspondant et un produit de réduction;

un chromogène qui réagit avec le produit de réduction en présence d'un agent, pour produire un composé coloré indiquant la présence d'un alcool dans ledit fluide biologique;

un inhibiteur concurrent de l'enzyme;

un agent servant à transformer le chromogène en composé coloré; et

un tampon pour maintenir un pH prédéterminé.

2. Composition solide selon la revendication 1, comprenant en outre un composé réductible qui réagit avec ledit alcool inférieur en présence de ladite enzyme pour produire ledit produit de réduction.

3. Composition solide selon l'une quelconque des revendications précédentes, dans laquelle ladite enzyme est l'alcool déshydrogénase.

4. Composition solide selon la revendication 2 ou 3, dans laquelle ledit composé réductible est le nicotinamide adénine dinucléotide.

5. Composition solide selon l'une quelconque des revendications précédentes, comprenant en outre un composé emprisonnant l'acétaldéhyde.

6. Composition solide selon la revendication 5, dans laquelle ledit composé emprisonnant l'acétaldéhyde est le semicarbazide.

7. Composition solide selon l'une quelconque des revendications précédentes, contenant en outre un composé sulfhydryle.

8. Composition solide selon l'une quelconque des revendications précédentes, dans laquelle ladite enzyme est l'alcool déshydrogénase et ledit inhibiteur concurrent est choisi parmi un pyrazole, un pyrazole halogéné, un alkylpyrazole et un stéroïde.

9. Composition solide selon l'une quelconque des revendications précédentes, dans laquelle ledit chromogène est choisi parmi un sel de tétrazolium, le 2,6-dichlorophénolindophénol et le bleu de méthylène, et ledit agent est choisi parmi la diaphorase, la phénazine et la thiazine.

10. Composition solide selon l'une quelconque des revendications précédentes, contenant en outre une protéine inerte.

11. Composition solide selon la revendication 1, dans laquelle ladite enzyme est l'alcool oxydase.

12. Composition solide selon la revendication 1 ou 11, dans laquelle ledit inhibiteur concurrent est l'urée.

13. Composition solide selon la revendication 1 ou 12, dans laquelle ledit agent est la peroxydase.

14. Composition solide selon la revendication 1 ou 13, dans laquelle ledit chromogène est choisi parmi le guaiacol, l'otoluidine et un dérivé de la benzidine.

15. Composition solide selon la revendication 11 ou 14, contenant en outre un protéine inerte.

16. Support inerte, utilisable dans la détermination d'alcool dans les fluides biologiques, comportant une première zone discrète imprégnée d'une première substance imprégnante pour doser un alcool et une seconde zone discète imprégnée d'une seconde substance imprégnante pour doser un alcool, ladite première substance imprégnante comprenant, en quantités efficaces, du nicotinamide adénine dinucléotide, de l'alcool déshydrogénase, un inhibiteur concurrent de l'alcool déshydrogénase, un réactif chromogène en présence d'un agent choisi parmi la diaphorase, la phénazine et la thiazine, l'un des composés parmi la diaphorase, la phénazine ou la thiazine, un composé emprisonnant l'acétaldéhyde, un tampon pour maintenir un pH prédéterminé et un composé stabilisant l'enzyme; et ladite seconde substance imprégnante comprenant, en quantités efficaces, de l'alcool oxydase, un inhibiteur concurrent de l'alcool oxydase, un chromogène réactif en présence de peroxydase, de la peroxydase, un tampon pour maintenir un pH prédéterminé et un composé stabilisant l'enzyme.